# EUROPÄISCHE PATENTSCHRIFT

(11) **EP 1 522 300 B1**
(45) Veröffentlichungstag und Bekanntmachung des Hinweises auf die Patenterteilung: **24.04.2013**
(21) Anmeldenummer: 03022402.6
(22) Anmeldetag: 06.10.2003
(51) Int. Cl.: A61G 13/12, A61B 5/103, A61B 5/107

(54) **Stützvorrichtung**
Support device
Dispositif de support

(43) Veröffentlichungstag der Anmeldung: 13.04.2005
(73) Patentinhaber: Zimmer GmbH, 8404 Winterthur (CH)
(72) Erfinder: Dominati, Simon-Paul, 13007 Marseille (FR); Grappiolo, Guido, Finale Ligure (SV) (IT)
(74) Vertreter: Manitz, Finsterwald & Partner GbR

(56) Entgegenhaltungen:
- EP-A- 0 244 274
- DE-A- 3 406 212
- DE-U- 20 213 711
- FR-A- 2 777 077
- US-A- 889 224
- US-A- 4 705 026
- US-A- 5 197 975

## Beschreibung

Die Erfindung betrifft eine Stützvorrichtung zur Abstützung eines in Laterallage liegenden Patienten am Becken.

US 889,224 beschreibt ein Instrument zum Anzeigen der Positionen der Wirbelknochen einer Wirbelsäule. Hierzu umfasst das Instrument einen vertikalen Ständer, an dem eine Vielzahl von horizontal verlaufenden Stäben angebracht ist, die jeweils vertikal und entlang ihrer Längsrichtung verstellbar sind, um jeweils einen Wirbelknochen abzutasten. Die Stäbe sind jeweils mit einer Längenskala versehen, um den Abstand des jeweiligen Wirbelknochens zum Ständer zu bestimmen.

FR 2 777 077 A 1 betrifft eine Vorrichtung, mit deren Hilfe die rückseitige Kontur einer Person bestimmt werden kann, um einen Lattenrost mit entsprechender Kontur herzustellen. Die Vorrichtung umfasst einen vertikal stehenden Block, an dem mehrere horizontal verlaufende Stangen mit T-förmigen Abschluss angebracht sind. Die Stangen sind ebenfalls jeweils vertikal und entlang ihrer Längsrichtung verstellbar. Der Block ist mit einer Stricheinteilung mit vertikalen Strichen versehen, an dem die Verschiebung der Stangen entlang ihrer Längsrichtung ablesbar ist.

EP 0 244 274 A 1 betrifft eine Vorrichtung zum Durchführen einer dreidimensionalen Skelettanalyse an einem Patienten, mit einem Vertikalelement, an dem ein Arm zur Stützung des Patienten höhenverschiebbar gelagert ist. Daneben ist eine Digitalisiereinrichtung vorgesehen, die mit dem Vertikalelement verbunden ist und mehrere starre Verbindungselemente und dazwischen angeordneten Messwertgeber sowie an ihrem freien Ende einen Digitalisierkopf umfasst, um die Lage eines Punktes oder einer Gruppe von Punkten am Körper des Patienten zu bestimmen.

Bei Hüftgelenkoperationen ist es für den Einbau einer künstlichen Hüftgelenkschale von entscheidender Bedeutung, dass der Operateur die Schalenachse räumlich in der richtigen Richtung zum Becken orientiert. Dies gilt sowohl für normale Operationstechniken als auch für computergestützte Operationstechniken (CAS; "Computer Aided Surgery").

CAS ermöglicht es, die Orientierung eines Knochens, beispielsweise des Beckens, im Raum zu bestimmen und während der Operation zu verfolgen. Hierzu ist an einem vom Operateur mit der Hand zu führenden Taster ein Dreibein fixiert, dessen freie Enden jeweils z.B. mit einer Kugel versehen sind. Die drei Kugeln definieren eine Ebene im Raum, deren Lage durch Bestimmen der relativen Lage der Kugeln zueinander mittels einer mit elektromagnetischer, z.B. infraroter Strahlung arbeitenden Nachweiseinrichtung bestimmt werden kann.

Mit der Spitze des Tasters können einzelne Punkte eines Knochens angetastet werden, wodurch die Lage dieser Knochenpunkte im Raum relativ zueinander über den Nachweis des Kugel-Dreibeins bestimmt werden kann. Dieser Vorgang wird auch als "Mapping" bezeichnet. Um die mittels des Tasters ertasteten Punkte auf dem Knochen eindeutig identifizieren zu können, werden auf einem Bildschirm die ertasteten Punkte an einem zuvor mittels Computertomografie (CT) oder durch eine mittels eines Tasters am jeweiligen Knochen vorgenommene Digitalisierung aufgenommenen Bild des Knochens ausgerichtet. Dies ist erforderlich, da für die Ertastung der einzelnen Punkte auf dem Knochen in der Praxis meist nur ein kleiner Bereich des gesamten Knochens zur Verfügung steht und der Operateur selbst nicht in der Lage ist, anhand dieser lediglich einen kleinen Bereich des gesamten Knochens repräsentierenden Tastpunkte die Orientierung des Knochens im Raum zu bestimmen. Durch Verknüpfen des zuvor aufgenommenen CT-Bildes mit den ertasteten Punkten kann jedoch mittels einer geeigneten Software eine Identifizierung der ertasteten Punkte in dem CT-Bild und damit eine Bestimmung der Orientierung des Knochens im Raum erfolgen. Derartige Systeme werden auch als Navigationssysteme bezeichnet, und für diese Vorgehensweise findet auch der Begriff "CT-gestütztes Mapping" Verwendung. Dies ist grundsätzlich bekannt, weshalb hierauf nicht näher eingegangen werden soll.

Ebenso ist es bekannt, ohne CT unter Zuhilfenahme von Röntgenbildern bestimmte markante Punkte des Knochens - soweit mit einem Taster erreichbar - anzutasten, um auf diese Weise die Raumlage des Knochens zu ermitteln.

Trotz dieser immerhin mit einem nicht unerheblichen technischen Aufwand verbundenen Unterstützung des Operateurs wird die Bestimmung der Orientierung von Knochen zunehmend dadurch erschwert, dass bei den heutigen Operationstechniken immer mehr mit möglichst kleinen Eingriffen gearbeitet wird, d.h. so genannte minimal invasive Techniken zum Einsatz kommen. Hierdurch wird das eigentliche Operationsfeld, an dem der Operateur mit dem Taster während des Mapping arbeiten kann, immer kleiner und die räumliche Orientierung am Patienten immer schwieriger.

Hinzu kommt, dass auch bei Verwendung der vorstehend erläuterten Navigationssysteme der Operateur nach wie vor eine Bezugsebene benötigt, um z.B. bei Hüftgelenkoperationen den korrekten Raumwinkel für die Achse der Hüftgelenkschale abzuleiten.

Hierbei ist der Operateur letztlich auf Schätzungen angewiesen: Bei Operationen mit in Rückenlage liegendem Patienten nimmt der Operateur an, dass eine Beckenebene, die durch die seitlichen, nach vorne vorstehenden Spitzen der Beckenknochen und durch das Schambein gebildet wird, parallel zur Auflagefläche des Operationstisches ausgerichtet ist, und dass die Längsachse des Operationstisches und des Patienten parallel zueinander verlaufen. Aus dieser angenommenen Lage des Patienten leitet der Operateur den Raumwinkel für die Achse der Hüftgelenkschale einschließlich ihrer Antetorsion ab. Dies gilt sowohl für die Bearbeitungswerkzeuge des Acetabulums als auch für die später eingesetzte Hüftgelenkschale selbst.

In der Laterallage liegt der Patient mit seiner gesunden Seite auf der Auflagefläche des Operationstisches auf, wobei für die aus den Spitzen der Beckenknochen und dem Schambein gebildete Beckenebene ein Winkel von 90° zur Auflagefläche angestrebt wird. Hierzu wird der Patient auf seiner Rückseite und auf seiner Vorderseite mit Stützkonstruktionen abgestützt, die eine sichere und unverrückbare Lage sicherstellen sollen. Auch hier ist der Operateur letztlich auf eine Schätzung angewiesen, da es für ihn praktisch unmöglich ist, die Lage der erwähnten Beckenebene an dem mit sterilen Tüchern abgedeckten Patienten zu bestimmen. Der Operateur kann sich nur darauf verlassen, dass der Winkel der Beckenebene zur Auflagefläche des Operationstisches tatsächlich etwa 90° beträgt.

Aufgabe der Erfindung ist es daher, Hüftgelenkoperationen zu erleichtern und insbesondere eine Möglichkeit zu schaffen, die Orientierung des Beckens im Raum möglichst genau zu bestimmen.

Die Lösung dieser Aufgabe erfolgt durch die Merkmale des Anspruchs 1.

Die Erfindung nutzt in vorteilhafter Weise den Umstand aus, dass das menschliche Becken markante Stellen aufweist, und stellt eine Vorrichtung zur Verfügung, welche diese markanten Stellen als Stützpunkte oder Abstützstellen für Stangen nutzt, d.h. erfindungsgemäß wurde erkannt, dass bestimmte Stellen des menschlichen Beckens als prädestinierte Stellen zum Erfassen der Raumlage des Beckens herangezogen werden können. Dabei werden die markanten Stellen des Beckens nicht lediglich als Abstützpunkte verwendet, sondern es wird gleichzeitig die Lage der Abstützpunkte mittels der Stützstangen vom Becken weg nach außen transportiert, so dass die einzelnen Lagen über die Erfassungsenden der Stützstangen erfasst und aufgrund der vorgegebenen bekannten Länge der Stützstangen ausgewertet werden können, um auf diese Weise relativ zum Becken die Achslage von Bearbeitungswerkzeugen wie z.B. von Kugelfräsern oder die Ausrichtung einer künstlichen Hüftgelenkpfanne exakt einstellen zu können.

Die erfindungsgemäße Stützvorrichtung kann an einem Operationstisch befestigt werden oder Bestandteil eines Operationstisches sein, so dass während der Operation die Lage der Stützstangen relativ zur Auflagefläche des Operationstisches bekannt ist. Die Verstellbarkeit der Stützstangen ermöglicht eine punktuelle Ertastung des Beckens des Patienten, wodurch die Enden der Stützstangen in eine räumliche Konfiguration gebracht werden können, welche die Beckenanatomie des Patienten widerspiegelt. Durch Ertasten vorgegebener charakteristischer Punkte oder Stellen auf dem Becken, deren Relativlage bekannt ist, ist die Lage des Beckens relativ zu den Stützstangen und damit zur Auflagefläche des Operationstisches eindeutig bestimmt. Wenn der Patient mit seinem Becken über die Anlagepunkte an den Enden der Stützstangen anliegt, ist der Patient somit nicht nur abgestützt, sondern die Stützstangen enthalten außerdem eine Information über die Lage des Beckens relativ zur Auflagefläche, wenn die Punkte oder Stellen des Beckens, an denen die Enden der Stützstangen anliegen, bekannt sind.

In vorteilhafter Weise kann die in den Stützstangen enthaltene Information mittels eines Navigationssystems "gelesen" werden, wie es eingangs beschrieben wurde, indem mittels des das Kugel-Dreibein tragenden Tasters die vom Patienten abgewandten freien Enden der Stützstangen angetastet werden. Aus der bekannten Länge der Stützstangen sowie aus der bekannten Lage der Stützstangen relativ zueinander kann auf diese Weise über die vom Patienten entfernten Enden der Stützstangen die Orientierung des Beckens des Patienten im Raum bestimmt werden.

Die Erfindung ermöglicht somit gewissermaßen eine Fernabtastung des Beckens, wobei die vom Patienten entfernten Enden der Stützstangen gemeinsam eine punktweise Abbildung des Beckens darstellen, die direkt zur Lagebestimmung des Beckens genutzt werden kann, wenn die Anlagepunkte der Stützstangen am Becken bekannt sind. Ein wesentlicher Vorteil der Erfindung besteht darin, dass sie die Durchführung von Hüftgelenkoperationen ermöglicht, ohne dass zuvor CT-Bilder des Beckens aufgenommen werden müssen. Der Grund hierfür ist, dass durch Antasten der vom Patienten entfernten Enden der Stützstangen eine indirekte Ertastung charakteristischer Punkte oder Stellen des Beckens ermöglicht wird, aus der direkt - d.h. ohne Identifizierung der Tastpunkte in einem CT-Bild - die Lage des Beckens im Raum, insbesondere relativ zur Auflagefläche des Operationstisches, abgeleitet werden kann. Dies ist insbesondere deshalb möglich, da in Frage kommende Stellen am Becken relativ weit auseinander liegen, was die Orientierungsbestimmung erleichtert.

Der Operateur kann mit den Stützstangen die mit seinen Fingern durch das Gewebe hindurch ertasteten charakteristischen Punkte des Beckens anfahren und deren Lage kontrollieren. Für die Erfassung der Lage des Beckens ist der Operateur also nicht auf freiliegende Bereiche des Beckens angewiesen. Daher können die Stützstangen an charakteristischen Stellen des Beckens angesetzt werden, die nicht erst unter Zuhilfenahme eines CT-Bildes am Becken identifiziert zu werden brauchen. Die Erfindung ermöglicht somit ein CT-freies Mapping, mit dem die Lage des Beckens im Raum eindeutig und mit hoher Genauigkeit bestimmt werden kann. Gerade bei dicken Patienten ist es in der Laterallage kaum möglich, den Beckenknochen an derjenigen Seite, mit welcher der Patient auf dem Operationstisch aufliegt, mit einem Taster anzufahren, wenn der Patient abgestützt und damit räumlich fixiert ist.

Ein weiterer Vorteil der erfindungsgemäßen Stützvorrichtung besteht darin, dass nicht nur die Neigung einer bestimmten Beckenebene zu einem grundsätzlich beliebigen Referenzsystem, z.B. zur Auflagefläche des Operationstisches, bestimmt werden kann, sondern auch die Mittellinie in dieser Beckenebene und deren Abweichung von einer zur Auflagefläche des Operationstisches parallel verlaufenden Ebene.

Ferner ist von Vorteil, dass der Körper des Patienten auf der Vorderseite vollständig mit sterilen Tüchern abgedeckt bleiben kann, da die Ertastung des Beckens und das Ansetzen massiver Stützstangen unter Zwischenlage der Tücher, d.h. durch die Tücher hindurch, erfolgen kann. Die erfindungsgemäße Stützvorrichtung braucht somit nicht in den sterilen Bereich einzudringen.

Mit Hilfe der Erfindung kann sich der Operateur oder ein Mapping-System während der Operation jederzeit ein Bild darüber machen, wie das Becken des Patienten im Raum orientiert ist.

Es sei an dieser Stelle ausdrücklich darauf hingewiesen, dass die erfindungsgemäße Stützvorrichtung in vorteilhafter Weise auch ohne ein Mapping- bzw. Navigationssystem verwendet werden kann. Durch die Auswahl geeigneter Anlagepunkte am Becken kann sich der Operateur auch dadurch ein Bild von der Lage des Beckens im Raum machen, dass er einfach an die vom Patienten entfernten Enden der Stützstangen eine Platte anlegt, deren Orientierung im Raum die Beckenorientierung widerspiegelt und somit für den Operateur sichtbar macht.

Vorteilhafte Ausführungsformen der Erfindung sind in den abhängigen Ansprüchen, der Beschreibung sowie der Zeichnung angegeben.

Wie bereits vorstehend zum Ausdruck gebracht, sind die Stützstangen vorzugsweise zur Ertastung der Beckenanatomie ausgebildet, insbesondere zur Ertastung vorgegebener charakteristischer Punkte oder Stellen auf dem Becken, wobei bevorzugt die charakteristischen Punkte oder Stellen eine vordere Beckenebene definieren, die zumindest näherungsweise senkrecht zur Median-Sagittalebene verläuft.

Vorzugweise dienen als Anlage- oder Tastpunkte oder -stellen die seitlichen, nach vorne vorstehenden Spitzen der Beckenknochen sowie das Schambein. Eine durch diese drei Punkte festgelegte Beckenebene verläuft senkrecht zur Median-Sagittalebene des Patienten. Diese charakteristischen Punkte des Beckens sind zur Bestimmung der Beckenorientierung besonders geeignet, so dass vorzugsweise die erfindungsgemäße Stützvorrichtung genau drei Stützstangen aufweist.

Eine einfache Anpassung der Stützvorrichtung an unterschiedliche körperliche Gegebenheiten wird erreicht, wenn gemäß einem weiteren bevorzugten Ausführungsbeispiel die Enden der Stützstangen jeweils mit einem auswechselbaren Druckstempel oder Stützfuß versehen sind. Es können mehrere Sätze unterschiedlich großer Druckstempel vorgesehen sein, insbesondere mehrere Sätze von Druckstempeln mit unterschiedlich großen Auflageflächen.

Ferner kann vorgesehen sein, dass die Stützstangen jeweils in Richtung ihrer Längserstreckung relativ zum Traggestell verstellbar sind. Hierdurch kann die wirksame Länge der Stützstangen, d.h. der Abstand zwischen dem Traggestell und dem Patienten, auf einfache Weise verändert werden.

In einem besonders bevorzugten Ausführungsbeispiel ist vorgesehen, dass die Stützstangen parallel zueinander verlaufen und die gleiche Länge aufweisen. Hierdurch kann direkt aus der Lage der vom Patienten entfernten Enden der Stützstangen auf die Orientierung des Beckens relativ zum Traggestell und damit zum Operationstisch geschlossen werden, da die freien Enden der Stützstangen gemeinsam eine direkte Abbildung des Beckens hinsichtlich der charakteristischen Punkte oder Stellen darstellen, an denen die Stützstangen mit ihren Tastenden am Patienten anliegen. Die von den Anlagepunkten am Becken festgelegte Ebene wird gewissermaßen längs der Stützstangen vom Patienten weg parallel versetzt. Die Orientierung dieser Beckenebene im Raum kann an den freien Enden der Stützstangen "abgelesen" werden.

Die Stützstangen können in zwei senkrecht aufeinander stehenden Richtungen relativ zueinander verstellbar sein.

Jede Stützstange ist an einem senkrecht zur Stützstange verlaufenden Tragarm angebracht, wobei die Tragarme an einem gemeinsamen Träger angebracht sind, der sich sowohl zu den Stützstangen als auch zu den Tragarmen senkrecht erstreckt.

Mittels Fixiereinrichtungen kann eine starre Raumkonfiguration herstellbar sein.

Insbesondere kann vorgesehen sein, dass die Stützstangen an den Tragarmen und/oder die Tragarme an dem Träger jeweils durch Festklemmen in Führungen fixierbar sind.

Die Erfindung betrifft außerdem einen Operationstisch mit wenigstens einer Auflagefläche für einen Patienten und mit einer erfindungsgemäßen Stützvorrichtung, wie sie vorstehend beschrieben wurde.

Die Stützvorrichtung kann lösbar am Operationstisch anbringbar oder ein Bestandteil des Operationstisches sein.

Vorzugsweise verlaufen die Stützstangen etwa parallel zur Auflagefläche des Operationstisches.

Ferner ist bevorzugt vorgesehen, dass die Stützstangen in parallel und senkrecht zur Auflagefläche verlaufenden Richtungen relativ zueinander verstellbar sind.

Des Weiteren wird erfindungsgemäß vorgeschlagen, dass ein auf der Auflagefläche liegender Patient zwischen der Stützvorrichtung und einer am Operationstisch angebrachten Gegenhalteeinrichtung einklemmbar ist. Hierdurch ist sichergestellt, dass der auf der Auflagefläche liegende Patient unverrückbar zwischen der Stützvorrichtung und der Gegenhalteeinrichtung festgehalten wird. Die Gegenhalteeinrichtung kann eine separate Vorrichtung sein, die lösbar am Operationstisch anbringbar oder ein Bestandteil des Operationstisches ist. Prinzipiell ist es auch möglich, die erfindungsgemäße Stützvorrichtung und eine Gegenhalteeinrichtung in eine einzige Vorrichtung zu integrieren, die entweder lösbar am Operationstisch anbringbar ist oder einen Bestandteil des Operationstisches bildet.

Die Gegenhalteeinrichtung kann wenigstens ein Stützkissen oder -polster umfassen, das bevorzugt derart ausgebildet ist, dass es oberhalb des Gesäßes des Patienten im Bereich der Lendenwirbel zur Anlage gebracht werden kann.

Die Erfindung betrifft außerdem ein Operationssystem mit wenigstens einer Stützvorrichtung oder einem Operationstisch nach einem der vorhergehenden Ansprüche und mit einer Erfassungseinrichtung zum Bestimmen der räumlichen Lage des Beckens des Patienten über die Erfassungsenden der Stützstangen der Stützvorrichtung, wobei die Erfassungseinrichtung eine Tasteinrichtung mit einem Taster, mittels welchem die Erfassungsenden der Stützstangen ertastbar sind, und eine einen Sender, einen Empfänger und eine Auswerteeinheit umfassende Nachweiseinrichtung aufweist, mittels welcher durch Auswerten von über den Sender ausgesandter, vom Taster reflektierter und mittels des Empfängers empfangener elektromagnetischer, insbesondere infraroter, Strahlung die räumliche Lage zumindest einer Abtastspitze, insbesondere zusätzlich die räumliche Lage einer durch die Abtastspitze verlaufenden Achse, des Tasters bestimmbar ist.

Die Erfindung wird im Folgenden beispielhaft unter Bezugnahme auf die Zeichnung beschrieben. Es zeigen:
- Fig. 1: eine perspektivische Ansicht einer Stützvorrichtung gemäß einer Ausführungsform der Erfindung,
- Fig. 2: die Stützvorrichtung von Fig. 1 in einer anderen perspektivischen Ansicht, und
- Fig. 3a, 3b: verschiedene Ansichten eines menschlichen Beckens, in die mögliche Anlagepunkte für die erfindungsgemäße Stützvorrichtung eingezeichnet sind.

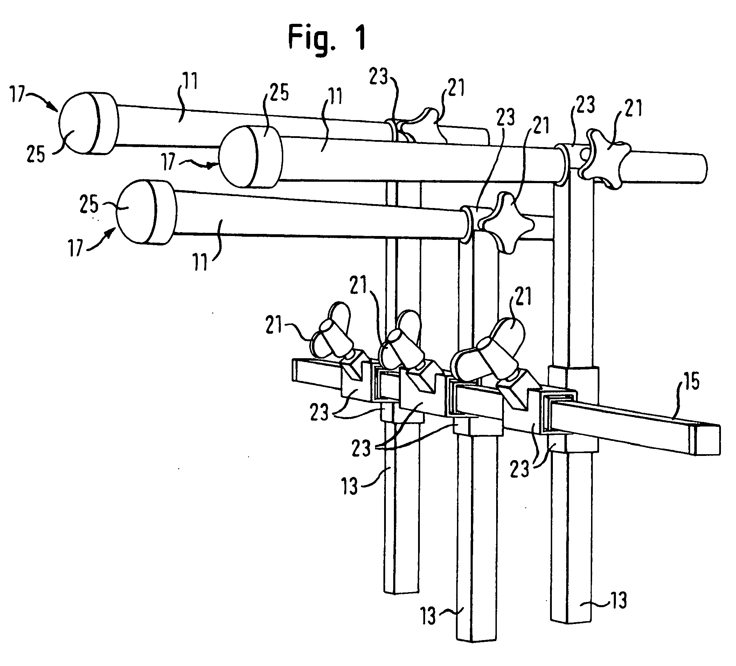

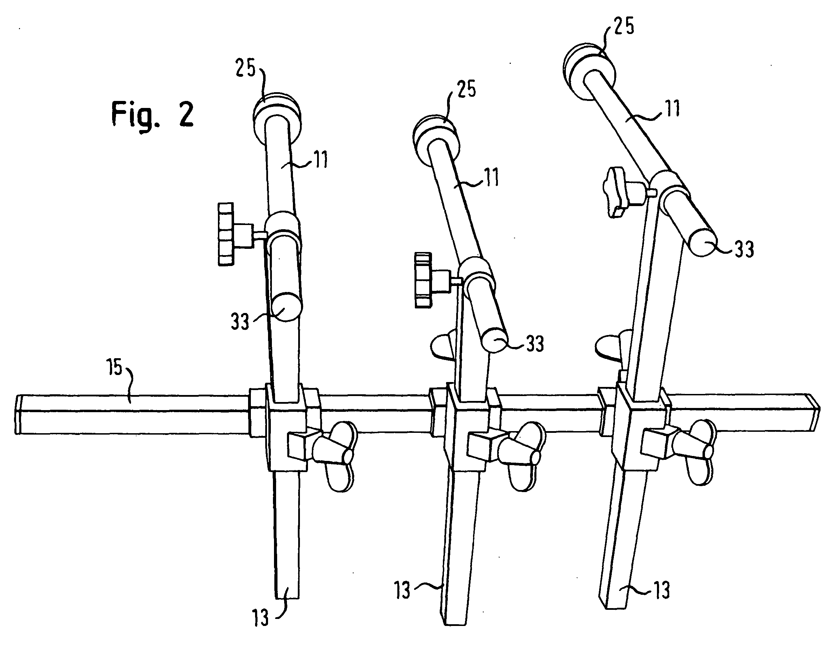

Die in den Fig. 1 und 2 dargestellte erfindungsgemäße Stützvorrichtung umfasst ein Traggestell mit einem gemeinsamen Träger 15 für drei parallele Tragarme 13, die jeweils durch einen zwei rechtwinklig zueinander verlaufende Führungen 23 umfassenden Verbinder mit dem Träger 15 verbunden sind.

Die entsprechend dem hier quadratischen Querschnitt des Trägers 15 und der Tragarme 13 ausgebildeten, hülsenartigen Führungen 23 sind auf das jeweilige Tragelement 15, 13 aufgeschoben. Mittels einer eine Flügelschraube umfassenden Fixiereinrichtung 21 sind die Tragelemente 15, 13 an der jeweiligen Führung 23 festklemmbar, so dass insgesamt eine starre Raumkonfiguration geschaffen werden kann.

Bei gelösten Fixiereinrichtungen 21 sind die Verbinder und damit die Tragarme 13 längs des Trägers 15 verschiebbar, wodurch die Abstände der parallel zueinander verlaufenden Tragarme 13 untereinander eingestellt werden können.

Die Tragarme 13 sind an ihrem einen freien Ende jeweils mit einer Führung 23 versehen, die einen kreisförmigen Querschnitt entsprechend dem kreisförmigen Querschnitt einer Stützstange 11 aufweist, welche durch die Führung 23 hindurch gesteckt und mittels einer eine Fixierschraube umfassenden Fixiereinrichtung 21 an der Führung 23 und damit am Tragarm 13 fixiert werden kann.

Mittels der insgesamt neun Fixiereinrichtungen 21 kann somit die den Träger 15, die Tragarme 13 sowie die Stützstangen 11 umfassende erfindungsgemäße Stützvorrichtung in eine starre Gesamt-Konfiguration gebracht werden, in welcher wie in einem kartesischen Koordinatensystem die Tragarme 13 senkrecht zum Träger 15 und die Stützstangen 11 sowohl zu den Tragarmen 13 als auch zum Träger 15 senkrecht verlaufen.

Auf einer Seite der Stützstangen 11 sind deren während der Operation als Tastenden 17 dienende freie Enden jeweils mit einem im Folgenden auch als Stützfuß bezeichneten Druckstempel 25 in Form einer aufsteckbaren Kappe versehen. Mittels dieser Stützfüße 25 kann die mit dem Patienten in Kontakt zu bringende Anlagefläche auf eine praktisch beliebige, von der Größe der Querschnittsfläche der jeweiligen Stützstange 11 auch abweichende Größe gebracht werden. Auf diese Weise kann die Größe der Auflagefläche der Stützfüße 25 gezielt an den jeweiligen Patienten und insbesondere an die Dicke des Fettgewebes des Patienten angepasst werden. Je dicker das Fettgewebe ist, desto kleiner wird die Auflagefläche der Druckstempel 25 gewählt, um an den jeweils gewünschten Anlagepunkt am Becken des Patienten herankommen zu können.

Die Anbringung der erfindungsgemäßen Stützvorrichtung an einem Operationstisch erfolgt beispielsweise über den Träger 15 mittels nicht dargestellter Fixier- und/oder Klemmeinrichtungen derart, dass sich die Stützstangen 11 parallel zur Auflagefläche des Operationstisches erstrecken. Da die Stützstangen 11 die gleiche Länge aufweisen, bilden die während der Operation vom Patienten entfernten Enden 33 (Fig. 2) der Stützstangen 11 eine Ebene, deren Lage im Raum mit der räumlichen Lage derjenigen Ebene übereinstimmt, die durch die drei Anlagepunkte am Becken des Patienten definiert wird, d.h. diese Beckenebene wird gewissermaßen aus dem Patienten herausprojiziert.

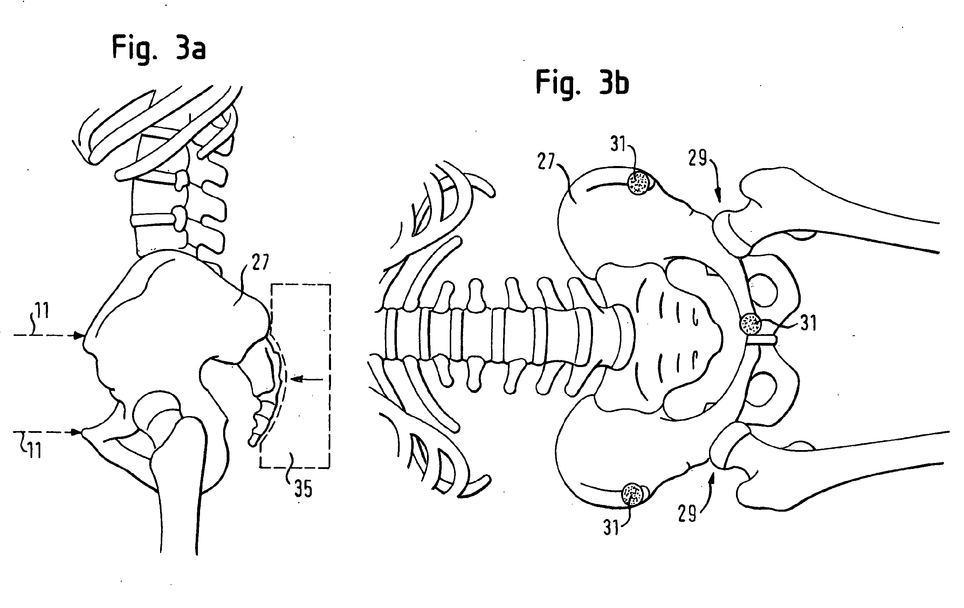

Die Fig. 3a und 3b zeigen anhand eines teilweise dargestellten Skelettes das Becken 27 eines eine Laterallage einnehmenden Patienten, wobei Fig. 3a eine Draufsicht von oben, d.h. senkrecht zur Auflagefläche des Operationstisches, und Fig. 3b eine Seitenansicht, d.h. parallel zur Auflagefläche des Operationstisches, darstellt.

Bevorzugte Anlagepunkte 31 auf der Vorderseite des Beckens 27 befinden sich gemäß Fig. 3b am Schambein und im Bereich der nach vorne vorstehenden seitlichen Spitzen des Beckens 27. Mit Hilfe der mit ihren Druckstempeln 25 an diesen Stellen 31 anliegenden Stützstangen 11, die in Fig. 3a lediglich schematisch dargestellt sind, kann eine senkrechte Orientierung der von den drei Anlagepunkten 31 definierten Beckenebene relativ zur Auflagefläche des Operationstisches sichergestellt werden.

Eine Unterstützung des Patienten von hinten erfolgt durch eine in Fig. 3a lediglich schematisch angedeutete Gegenhalteeinrichtung 35 in Form eines Polsters oder Kissens, das ebenfalls am Operationstisch angebracht ist, so dass der Patient zwischen den Stützstangen 11 einerseits und der Gegenhalteeinrichtung 35 andererseits unverrückbar fixiert ist, wie es durch die Pfeile in Fig. 3a angedeutet ist.

Die Vorgehensweise bei der Verwendung der erfindungsgemäßen Stützvorrichtung im Rahmen einer Hüftgelenkoperation ist wie folgt:

Zunächst wird der Patient provisorisch in der Laterallage derart gelagert, dass die durch die anschließend mittels der Stützstangen 11 zu ertastende Beckenebene möglichst genau einen Winkel von 90° mit der Auflagefläche des Operationstisches einschließt. Anschließend werden die äußeren Bereiche des Patienten mit sterilen Tüchern abgedeckt. Der Patient wird hierbei noch manuell gestützt.

Anschließend werden die drei Druckstempel 25 der Stützstangen 11 an die drei Anlagepunkte 31 herangeführt, indem die Tragarme 13 längs des Trägers 15 und die Stützstangen 11 in Richtung ihrer Längsachse relativ zu den Tragarmen 13 verstellt werden. Dabei wird die Lage der Druckstempel 25 in Bezug auf die gewünschten Anlagepunkte 31 durch die sterilen Tücher und durch das Gewebe das Patienten hindurch ertastet und kontrolliert.

Mehr oder weniger gleichzeitig wird von hinten oberhalb des Gesäßes die Gegenhalteeinrichtung 35, z.B. ein Druckkissen, angesetzt, wodurch die Anlagepunkte 31 des Beckens 27 an die Stützfüße 25 angedrückt werden.

Da die Stützstangen 11 in dem hier beschriebenen Ausführungsbeispiel die gleiche Länge aufweisen, ist die von den drei Anlagepunkten 31 definierte Beckenebene parallel verschoben an den vom Patienten entfernten freien Enden 33 der Stützstangen 11 ablesbar oder abtastbar. Dies kann - muss aber nicht - mit Hilfe eines CT-freien Mapping-Verfahrens unter Verwendung einer Nachweiseinrichtung erfolgen, wie es vorstehend erläutert wurde.

Dem Operateur ist nunmehr die räumliche Orientierung des Beckens 27 relativ zur Auflagefläche des Operationstisches bekannt, so dass hiervon ausgehend der korrekte Raumwinkel für die Achse eines Kugelfräsers oder einer Hüftgelenkschale des zu operierenden Hüftgelenkes 29 (Fig. 3b) sicher und zuverlässig abgeleitet werden kann.

### Bezugszeichenliste

| | |
|---|---|
| 11 | Stützstange |
| 13 | Tragarm |
| 15 | Träger |
| 17 | Tastende |
| 21 | Fixiereinrichtung |
| 23 | Führung |
| 25 | Druckstempel, Stützfuß |
| 27 | Becken |
| 29 | Hüftgelenk |
| 31 | Anlagepunkt |
| 33 | freies Ende der Stützstange, Erfassungsende |
| 35 | Gegenhalteeinrichtung |

## Patentansprüche

1. Stützvorrichtung zur Abstützung eines in Laterallage liegenden Patienten am Becken sowie zur Erfassung der Raumlage des Beckens während einer Operation, mit einem Traggestell bestehend aus einer Mehrzahl von Tragarmen (13) und einem gemeinsamen Träger (15), und mit einer Mehrzahl von jeweils eine bekannte vorgegebene Länge aufweisenden Stützstangen (11) mit Tastenden und mit den Tastenden entgegengesetzten, als Erfassungsenden diendenden freien Enden, insbesondere genau drei Stützstangen (11), die relativ zueinander verstellbar sind, so dass verschiedene räumliche Konfigurationen der bei Benutzung dem Patienten zugewandten Tastenden (17) der Stützstangen (11) einstellbar sind, wobei durch die räumliche Lage der Stützpunkte (31), an welchen die Stützstangen (11) mit ihren Tastenden (17) bei Benutzung am Patienten abgestützt sind, eine Beckenebene erfasst werden kann, die über die freien Enden (33) der Stützstangen (11) erfassbar ist, wobei jede Stützstange (11) an einem senkrecht zur Stützstange (11) verlaufenden Tragarm (13) verstellbar angebracht ist, wobei die Tragarme (13) an dem gemeinsamen Träger (15) verstellbar angebracht sind, der sich sowohl zu den Stützstangen (11) als auch zu den Tragarmen (13) senkrecht erstreckt.

2. Stützvorrichtung nach Anspruch 1,
**dadurch gekennzeichnet,**
**dass** die Stützstangen (11) zur Ertastung der Beckenanatomie ausgebildet sind.

3. Stützvorrichtung nach Anspruch 1 oder 2,
**dadurch gekennzeichnet,**
**dass** die Stützstangen (11) zur Ertastung vorgegebener charakteristischer Punkte oder Stellen (31) auf dem Becken (27), insbesondere auf der Vorderseite des Beckens, ausgebildet sind, wobei vorzugsweise die charakteristischen Punkte oder Stellen (31) eine vordere Beckenebene definieren, die bevorzugt zumindest näherungsweise senkrecht zur Median-Sagittalebene verläuft.

4. Stützvorrichtung nach einem der vorhergehenden Ansprüche,
**dadurch gekennzeichnet,**
**dass** die Stützstangen (11) derart ausgebildet sind, dass das Becken (27) des Patienten mittels der Stützstangen (11) hinsichtlich vorgegebener charakteristischer Punkte oder Stellen (31) auf dem Becken (27), insbesondere auf der Vorderseite des Beckens (27), abbildbar ist.

5. Stützvorrichtung nach einem der vorhergehenden Ansprüche,
**dadurch gekennzeichnet,**
**dass** die Tastenden (17) der Stützstangen (11) jeweils mit einem auswechselbaren Druckstempel oder Stützfuß (25) versehen sind.

6. Stützvorrichtung nach Anspruch 5,
**dadurch gekennzeichnet,**
**dass** mehrere Sätze unterschiedlich großer Druckstempel bzw. Stützfüße (25) vorgesehen sind.

7. Stützvorrichtung nach einem der vorhergehenden Ansprüche,
**dadurch gekennzeichnet,**
**dass** die Stützstangen (11) jeweils in Richtung ihrer Längserstreckung relativ zum Traggestell (13, 15) verstellbar sind.

8. Stützvorrichtung nach einem der vorhergehenden Ansprüche,
**dadurch gekennzeichnet,**
**dass** die Stützstangen (11) parallel zueinander verlaufen.

9. Stützvorrichtung nach einem der vorhergehenden Ansprüche,
**dadurch gekennzeichnet,**
**dass** die Stützstangen (11) die gleiche Länge aufweisen.

10. Stützvorrichtung nach einem der vorhergehenden Ansprüche,
**dadurch gekennzeichnet,**
**dass** die Stützstangen (11) jeweils einen kreisförmigen Querschnitt aufweisen.

11. Stützvorrichtung nach einem der vorhergehenden Ansprüche,
**dadurch gekennzeichnet,**
**dass** die Stützstangen (11) in zwei senkrecht aufeinander stehenden Richtungen relativ zueinander verstellbar sind.

12. Stützvorrichtung nach einem der vorhergehenden Ansprüche,
**dadurch gekennzeichnet,**
**dass** die Stützstangen (11) an den Tragarmen (13) und/oder die Tragarme (13) an dem Träger (15) jeweils durch Festklemmen in Führungen (23) fixierbar sind.

13. Stützvorrichtung nach einem der vorhergehenden Ansprüche,
**dadurch gekennzeichnet,**
**dass** mittels Fixiereinrichtungen (21) eine starre Raumkonfiguration herstellbar ist.

14. Stützvorrichtung nach einem der vorhergehenden Ansprüche,
**dadurch gekennzeichnet,**
**dass** das Traggestell (13, 15) an einem Operationstisch befestigbar ist.

15. Stützvorrichtung nach einem der Ansprüche 1 bis 13,
**dadurch gekennzeichnet,**
**dass** das Traggestell (13, 15) ein Bestandteil eines Operationstisches ist.

16. Operationstisch mit einer Auflagefläche für einen Patienten und mit wenigstens einer Stützvorrichtung nach einem der vorhergehenden Ansprüche.

17. Operationstisch nach Anspruch 16,
**dadurch gekennzeichnet,**
**dass** die Stützvorrichtung lösbar am Operationstisch anbringbar ist.

18. Operationstisch nach Anspruch 16,
**dadurch gekennzeichnet,**
**dass** die Stützvorrichtung ein Bestandteil des Operationstisches ist.

19. Operationstisch nach einem der Ansprüche 16 bis 18,
**dadurch gekennzeichnet,**
**dass** die Stützstangen (11) etwa parallel zur Auflagefläche verlaufen.

20. Operationstisch nach einem der Ansprüche 16 bis 19,
**dadurch gekennzeichnet,**
**dass** die Stützstangen (11) in parallel und senkrecht zur Auflagefläche verlaufenden Richtungen relativ zueinander verstellbar sind.

21. Operationstisch nach einem der Ansprüche 16 bis 20,
**dadurch gekennzeichnet,**
**dass** ein auf der Auflagefläche liegender Patient zwischen der Stützvorrichtung und einer am Operationstisch angebrachten Gegenhalteeinrichtung (35) einklemmbar ist.

22. Operationstisch nach Anspruch 21,
**dadurch gekennzeichnet,**
**dass** die Gegenhalteeinrichtung (35) wenigstens ein Stützkissen oder -polster umfasst.

23. Operationssystem mit wenigstens einer Stützvorrichtung nach einem der Ansprüche 1 bis 15 oder einem Operationstisch nach einem der Ansprüch 16 bis 22 und mit einer Erfassungseinrichtung zum Bestimmen der räumlichen Lage des Beckens des Patienten über die Erfassungsenden (33) der Stützstangen (11) der Stützvorrichtung, wobei die Erfassungseinrichtung eine Tasteinrichtung mit einem Taster, mittels welchem die Erfassungsenden (33) der Stützstangen (11) ertastbar sind, und eine einen Sender, einen Empfänger und eine Auswerteeinheit umfassende Nachweiseinrichtung aufweist, mittels welcher durch Auswerten von über den Sender ausgesandter, vom Taster reflektierter und mittels des Empfängers empfangener elektromagnetischer, insbesondere infraroter, Strahlung die räumliche Lage zumindest einer Abtastspitze, insbesondere zusätzlich die räumliche Lage einer durch die Abtastspitze verlaufenden Achse, des Tasters bestimmbar ist.

## Claims

1. A support apparatus for the support at the pelvis of a patient lying in the lateral position and for detecting the spatial position of the pelvis during surgery having a support frame comprising a plurality of support arms (13) and a common support (15) and having a plurality of support rods (11) having probe ends and free ends opposite the probe ends and serving as detection ends, in particular precisely three support rods (11), with the support rods each having a known predefined length and being adjustable relative to one another, such that different spatial configurations of the probe ends (17) of the support rods (11) facing the patient in use can be set, wherein a pelvic plane can be detected by the spatial position of the support points (31) at which the probe ends (17) of the support rods (11) are supported at the patient in use, said pelvic plane being able to be detected via the free ends (33) of the support rods (11), wherein each support rod (11) is adjustably attached to a support arm (13) extending perpendicular to the support rod (11), wherein the support arms (13) are adjustably attached to the common support (15) which extends perpendicular both to the support rods (11) and to the support arms (13).

2. A support apparatus in accordance with claim 1,
**characterized in that**
the support rods (11) are configured for the probing of the pelvic anatomy.

3. A support apparatus in accordance with claim 1 or claim 2,
**characterized in that**
the support rods (11) are configured for the probing of predefined characteristic points or positions (31) on the pelvis (27), in particular on the anterior side of the pelvis, with the characteristic points or positions (31) preferably defining an anterior pelvic plane which preferably extends at least approximately perpendicular to the median sagittal plane.

4. A support apparatus in accordance with any one of the preceding claims,
**characterized in that**
the support rods (11) are configured such that the pelvis (27) of the patient can be mapped by means of the support rods (11) with respect to predefined characteristic points or positions (31) on the pelvis (27), in particular on the anterior side of the pelvis (27).

5. A support apparatus in accordance with any one of the preceding claims,
**characterized in that**
the probe ends (17) of the support rods (11) are each provided with a replaceable pressure piston or support foot (25).

6. A support apparatus in accordance with claim 5,
**characterized in that**
a plurality of sets of differently sized pressure pistons or support feet (25) are provided.

7. A support apparatus in accordance with any one of the preceding claims,
**characterized in that**
the support rods (11) are each adjustable relative to the support frame (13, 15) in the direction of their longitudinal extents.

8. A support apparatus in accordance with any one of the preceding claims,
**characterized in that**
the support rods (11) extend in parallel to one another.

9. A support apparatus in accordance with any one of the preceding claims,
**characterized in that**
the support rods (11) have the same length.

10. A support apparatus in accordance with any one of the preceding claims,
**characterized in that**
the support rods (11) each have a circular cross-section.

11. A support apparatus in accordance with any one of the preceding claims,
**characterized in that**
the support rods (11) are adjustable relative to one another in two directions standing perpendicular to one another.

12. A support apparatus in accordance with any one of the preceding claims,
**characterized in that**
the support rods (11) are fixable to the support arms (13) and/ or the support arms (13) are fixable to the support (15) in each case by clamping in guides (23).

13. A support apparatus in accordance with any one of the preceding claims,
**characterized in that**
a rigid spatial configuration can be established by means of fixing devices (21).

14. A support apparatus in accordance with any one of the preceding claims,
**characterized in that**
the support frame (13, 15) can be fastened to an operating table.

15. A support apparatus in accordance with one of the claims 1 to 13,
**characterized in that**
the support frame (13, 15) is a component of an operating table.

16. An operating table having a support surface for a patient and having at least one support apparatus in accordance with any one of the preceding claims.

17. An operating table in accordance with claim 16,
**characterized in that**
the support apparatus can be releasably attached to the operating table.

18. An operating table in accordance with claim 16,
**characterized in that**
the support apparatus is a component of the operating table.

19. An operating table in accordance with any one of the claims 16 to 18,
**characterized in that**
the support rods (11) extend approximately in parallel to the support surface.

20. An operating table in accordance with any one of the claims 16 to 19,
**characterized in that**
the support rods (11) are adjustable relative to one another in directions extending in parallel and perpendicular to the support surface.

21. An operating table in accordance with any one of the claims 16 to 20,
**characterized in that**
a patient lying on the support surface can be clamped between the support apparatus and a counter-holding device (35) attached to the operating table.

22. An operating table in accordance with claim 21,
**characterized in that**
the counter-holding device (35) includes at least one support cushion or support pad.

23. An operating system having at least one support apparatus in accordance with any one of the claims 1 to 15 or an operating table in accordance with any one of the claims 16 to 22 and having a detection device for the determination of the spatial position of the pelvis of the patient via the detection ends (33) of the support rods (11) of the support apparatus, wherein the detection device has a probe device with a probe by means of which the detection ends (33) of the support rods (11) can be probed, and has an indication device which includes a transmitter, a receiver and an evaluation unit and by means of which the spatial position of at least one probe tip of the probe, in particular additionally the spatial position of an axis extending through the probe tip, can be determined by evaluation of electromagnetic radiation, in particular infrared radiation, transmitted via the transmitter, reflected from the probe and received by means of the receiver.

## Revendications

1. Dispositif de support pour soutenir un patient reposant en position latérale au niveau du bassin ainsi que pour détecter la position du bassin dans l'espace pendant une opération, comprenant un châssis support formé d'une pluralité de bras porteurs (13) et d'un support commun (15), et comprenant une pluralité de barres de soutien (11) présentant chacune une longueur prédéterminée connue avec des extrémités de palpage, et avec des extrémités libres opposées aux extrémités de palpage et servant d'extrémités de détection, en particulier exactement trois barres de soutien (11), qui sont déplaçables les unes par rapport aux autres, de sorte qu'il est possible de régler différentes configurations dans l'espace des extrémités de palpage (17), tournées vers le patient lors de l'utilisation, des barres de soutien (11), et grâce à la position dans l'espace des points de soutien (31), au niveau desquels les barres de soutien (11) sont soutenues sur le patient avec leurs extrémités de palpage (17) lors de l'utilisation, il est possible de détecter un plan de bassin, lequel peut être détecté via les extrémités libres (33) des barres de soutien (11), et chaque barre de soutien (11) est montée de façon réglable sur un bras porteur (13) s'étendant perpendiculairement à la barre de soutien (11), dans lequel les bras porteurs (13) sont montés de façon réglable sur le support commun (15), lequel s'étend perpendiculairement à la fois aux barres de soutien (11) et aux bras porteurs (13).

2. Dispositif de support selon la revendication 1,
**caractérisé en ce que** les barres de soutien (11) sont réalisées pour palper l'anatomie du bassin.

3. Dispositif de support selon la revendication 1 ou 2,
**caractérisé en ce que** les barres de soutien (11) sont réalisées pour palper des points ou des emplacements caractéristiques prédéterminés (31) sur le bassin (27), en particulier sur le côté antérieur du bassin, et les points ou les emplacements caractéristiques (31) définissent de préférence un plan de bassin antérieur qui s'étend de préférence au moins approximativement perpendiculairement au plan sagittal médian.

4. Dispositif de support selon l'une des revendications précédentes,
**caractérisé en ce que** les barres de soutien (11) sont réalisées de telle façon que le bassin (27) du patient peut faire l'objet d'une image au moyen des barres de soutien (11) pour ce qui concerne des points ou des emplacements (31) caractéristiques prédéterminés sur le bassin (27), en particulier sur le côté antérieur du bassin (27).

5. Dispositif de support selon l'une des revendications précédentes,
**caractérisé en ce que** les extrémités de palpage (17) des barres de soutien (11) sont dotées chacune d'un piston presseur ou d'un pied de soutien (25) interchangeable.

6. Dispositif de support selon la revendication 5,
**caractérisé en ce qu'**il est prévu plusieurs jeux de pistons presseurs ou de pieds de soutien (25) de tailles différentes.

7. Dispositif de support selon l'une des revendications précédentes,
**caractérisé en ce que** les barres de soutien (11) sont déplaçables respectivement en direction de leur extension longitudinale par rapport au châssis porteur (13, 15).

8. Dispositif de support selon l'une des revendications précédentes,
**caractérisé en ce que** les barres de soutien (11) s'étendent parallèlement les unes aux autres.

9. Dispositif de support selon l'une des revendications précédentes,
**caractérisé en ce que** les barres de soutien (11) ont la même longueur.

10. Dispositif de support selon l'une des revendications précédentes,
**caractérisé en ce que** les barres de soutien (11) présentent chacune une section de forme circulaire.

11. Dispositif de support selon l'une des revendications précédentes,
**caractérisé en ce que** les barres de soutien (11) sont déplaçables les unes par rapport aux autres dans deux directions perpendiculaires l'une à l'autre.

12. Dispositif de support selon l'une des revendications précédentes,
**caractérisé en ce que** les barres de soutien (11) sont susceptibles d'être fixées sur les bras porteurs (13) et/ou les bras porteurs (13) sont susceptibles d'être fixés sur le support (15) respectivement par coincement dans les guidages (23).

13. Dispositif de support selon l'une des revendications précédentes,
**caractérisé en ce qu'**il est possible d'établir une configuration rigide dans l'espace au moyen de systèmes de fixation (21).

14. Dispositif de support selon l'une des revendications précédentes,
**caractérisé en ce que** le châssis porteur (13, 15) est susceptible d'être fixé sur une table d'opération.

15. Dispositif de support selon l'une des revendications 1 à 13,
**caractérisé en ce que** le châssis porteur (13, 15) est une partie constitutive d'une table d'opération.

16. Table d'opération présentant une surface d'appui pour un patient, et au moins un dispositif de support selon l'une des revendications précédentes.

17. Table d'opération selon la revendication 16,
**caractérisée en ce que** le dispositif de support est susceptible d'être monté de façon détachable sur la table d'opération.

18. Table d'opération selon la revendication 16,
**caractérisée en ce que** le dispositif de support est une partie constitutive de la table d'opération.

19. Table d'opération selon l'une des revendications 16 à 18, **caractérisée en ce que** les barres de soutien (11) s'étendent approximativement parallèlement à la surface d'appui.

20. Table d'opération selon l'une des revendications 16 à 19, **caractérisée en ce que** les barres de soutien (11) sont déplaçables les unes par rapport aux autres dans des directions s'étendant parallèlement et perpendiculairement à la surface d'appui.

21. Table d'opération selon l'une des revendications 16 à 20, **caractérisée en ce qu'**un patient reposant sur la surface d'appui est susceptible d'être coincé entre le dispositif de support et un système de maintien antagoniste (35) monté sur la table d'opération.

22. Table d'opération selon la revendication 21,
**caractérisée en ce que** le système de maintien antagoniste (35) comprend au moins un coussin ou un rembourrage de soutien.

23. Système d'opération comprenant au moins un dispositif de support selon l'une des revendications 1 à 15 ou une table d'opération selon l'une des revendications 16 à 22, et comprenant un moyen de détection pour déterminer la position dans l'espace du bassin du patient via les extrémités de détection (33) des barres de soutien (11) du dispositif de support, dans lequel le moyen de détection comprend un dispositif de palpage avec un palpeur au moyen duquel les extrémités de détection (33) des barres de soutien (11) peuvent être palpées, et un système de mise en évidence comprenant un émetteur, un récepteur et une unité d'évaluation, au moyen duquel, par évaluation d'un rayonnement électromagnétique, en particulier infrarouge, émis par l'émetteur, réfléchi par le palpeur et reçu au moyen du récepteur, la position dans l'espace d'au moins une pointe de palpage, en particulier additionnellement la position dans l'espace d'un axe, s'étendant à travers la pointe de palpage, du palpeur, peut être déterminée.
